# EUROPEAN PATENT APPLICATION

(11) **EP 4 275 654 A1**
(43) Date of publication of application: **15.11.2023**
(21) Application number: 22020226.1
(22) Date of filing: 13.05.2022
(51) Int. Cl.: A61F 2/24

(54) **TRANSCATHETER TREATMENT OF ATRIOVENTRICULAR VALVES, TO TREAT LEAFLET PROLAPSE**

(71) Applicant: Verine, Vitali, 1207 Geneva (CH)
(72) Inventor: Verine, Vitali, 1207 Geneva (CH)

(57) **Abstract**

Method and apparatus for treating prolapse of an atrioventricular valve. An implant (30) is delivered by catheter (32) into the heart, the implant configured to attach to a native chordae tendineae (20) and to shorten the functional length of the native chordae tendineae.

## Description

### Field of the Invention

The present invention relates to the field of transcatheter treatment of atrio-ventricular cardiac valves. Some non-limiting aspects are directed to treating prolapse of native valve leaflets.

### Background to the Invention

The mammalian heart has two atrio-ventricular (A-V) valves which regulate blood flow internally between the atrium and ventricle. Correct atrio-ventricular valve function is important to maintain efficient pumping of blood by the heart. In a healthy heart, each atrio-ventricular valve opens during diastole to admit blood from the atrium to the ventricle, and closes during systole to prevent backflow. However, many patients suffer from atrio-ventricular valve insufficiency, when the leaflets fail to close properly, which is a life-degrading condition, and can be life-threatening. Blood regurgitation through the valve reduces the efficiency of the heart, requiring the heart muscle to work harder to compensate. Not only does regurgitation reduce flow of blood out of the heart circulating to organs, it exposes the atrium to an abnormal increase in blood pressure. Excessive atrial pressure can negatively impact the atria and incoming veins, and also obstruct free flow of blood into the heart.

Insufficiency of an atrio-ventricular valve can have many causes. A frequent cause is prolapse of one or both native leaflets. If the patient is suitable for open-heart surgery, prolapse can be treated by various surgical techniques, for example, mitral ring annuloplasty, chordae shortening by surgically burying the chordae in a papillary muscle trench, and more recently surgical implantation of artificial chordae. However, a large number of patients are not suitable for open heart surgery, for example, due to age or frailty or other risk factors and comorbidities.

Minimally invasive treatment of atrio-ventricular valve prolapse has long been sought after, but current technologies have significant limitations. Minimally invasive options include transcatheter implantation of a prosthetic replacement valve, and transcatheter implantation of devices that modify the native valve apparatus, such as leaflet clips, leaflet coaptation surfaces, artificial chordae and annuloplasty devices. However, current replacement atrio-ventricular valves are expensive, relatively large in size with special anchoring, and require a large diameter catheter that limits access to a transapical route. Leaflet clips and coaptation modifying devices restrict the ability of the valve to open fully, and so the treatment may have undesirable secondary effects. Artificial chordae are problematic because they have to be anchored to native tissue at either end which is difficult to achieve reliably using only catheter tools. Also, unlike native chordae, it is not possible to attach artificial chordae to mobile regions of the leaflet. Therefore, artificial chordae do not perform the full function of native chordae. Transcatheter annuloplasty devices can improve valve function within limits, but are best used in combination with another treatment.

### Summary of the Invention

It would be desirable to address and/or mitigate one or more of the above issues.

Broadly speaking, a first aspect of the invention provides apparatus for treating prolapse of an atrioventricular valve, the apparatus comprising an implant deliverable by catheter, the implant configured to attach to a native chordae tendineae and to shorten the functional length of the chordae tendineae.

As used herein the term "chordae tendineae", or hereafter "chordae" refers to any of the chords of tissue that connect the native atrioventricular leaflets to the papillary muscles. Optionally, the implant is attachable to an individual native chordae, and to shorten the functional length of the respective individual chordae.

By shortening the functional length, an existing native chordae can better limit the range of movement of the native leaflet and enable leaflet prolapse to be corrected, without many of the limitations and inconveniences of prior art techniques discussed above. Shortening the functional length can also maintain or re-establish correct anatomical function of the or a native chordae, which is a consideration overlooked by many prior art techniques. For example, the native chordae can maintain or re-establish tension force between the native leaflets and the papillary muscles of the ventricle, which may be beneficial to heart function and to ventricle modelling behaviour.

In some embodiments, the implant is configured to shorten the functional length by diverting a portion of the native chordae along a circuitous path. The circuitous path may optionally be a path into the implant, for example, into an interior cavity of the implant and/or into a space bound at least partly by the implant. Optionally, the implant defines at least partly the circuitous path along which the diverted portion of the native chordae extends, optionally at least a majority of the circuitous path, optionally substantially the entirety of the circuitous path. By using the implant to define the circuitous path, at least the majority of the diverted portion of the native chordae is controlled and supported by the implant, and not left loose to flap or flutter in an uncontrolled way, which may otherwise be unnatural and/or undesirable from a surgical point of view.

In some embodiments, the apparatus comprises a hook element for hooking the native chordae. The hook may form part of the implant or part of a delivery catheter for delivering the implant to a target site in the heart. The hook may be configured for pulling the native chordae in a proximal direction, the proximal direction being defined with respect to the catheter. Additionally or alternatively, the hook may be configured for pulling a portion of the native chordae into and/or through a portion of the implant, for example, into and/or through (i) a cavity of the implant, and/or (ii) a space bounded by the implant.

The implant may be configured to change configuration between a non-deployed configuration for delivery to a target site in the heart, and a deployed configuration for attaching to a native chordae.

For example, the implant may comprise a first portion and second portion, one portion being configured to deploy around or with respect to, and/or fold around, the other when the implant changes from the non-deployed configuration to the deployed configuration.

Additionally or alternatively, the implant may be configured to compress the native chordae tendineae and/or compress around the native chordae tendineae, when the implant changes from the non-deployed configuration to the deployed configuration.

In some embodiments, the implant comprises a curved surface configured for defining an at least partly curved circuitous path for the native chordae tendineae. Optionally, the curved surface defines a majority of the circuitous path as a curved path. Providing a curved path can provide atraumatic contact with the native chordae tissue, as well as adding to the path length.

Additionally or alternatively, the implant comprises an elongate clamping surface configured to provide spread clamping contact, spread along a length of a portion of a native chordae. The elongate clamping surface may optionally be any one or more of: curved; linear; curvilinear; defined by interlocking toothed profiles.

Additionally or alternatively to any of the above, the implant may have a streamlined shape, in some examples a bead-shape (optionally a spherical shape). A streamlined shape may reduce any tendency for the implant to be influenced by blood flow around the implant.

Additionally or alternatively, the implant may have a (e.g. maximum) transverse dimension (e.g. diameter) of less than 6mm, optionally less than 5mm, optionally less than 4mm. For example, the dimension may be in the range of 2 to 5mm, optionally 2 to 4mm, optionally 2-3mm.

Additionally or alternatively to any of the above, the implant may comprise, and/or be made of, one or more lightweight materials, such as one or more of: titanium; titanium-containing alloy; polyether ether ketone (PEEK); pyloric carbon. Lightweight materials may reduce loads on the native chordae, and may also reduce risk of the native chordae becoming totally disconnected at both ends even if a diseased or damaged native chordae should rupture at one point.

Additionally or alternatively to any of the above, the implant may comprise a wire mesh and/or braid structure. Such a structure can combine flexibility with a sufficiently strong predefined shape, for example in the deployed configuration, to remain securely and atraumatically attached to a native chordae.

Additionally or alternatively to any of the above, the implant may comprise one or more surfaces defining at least partly a channel for accommodating and pressing on a native chordae extending therethrough. At least one surface may comprise and/or be coated with and/or carry (e.g. as a liner), a compressible and/or flowable material able to conform at least partly around the profile of the native chordae while frictionally engaging against the native chordae. Example materials include silicone, polyurethane foam, and biocompatible rubbers. Such an arrangement can provide a secure yet atraumatic engagement with a native chordae.

A closely related second aspect of the invention provides an implant deliverable by catheter for treating prolapse of an atrioventricular valve (for example, by shortening a functional length of a native chordae), the implant comprising a hook for hooking a native chordae, the hook positioned internally with respect to a portion of the implant.

A closely related third aspect of the invention provides an implant deliverable by catheter for treating prolapse of an atrioventricular valve (for example, by shortening a functional length of a native chordae), the implant comprising a hook for hooking a native chordae, the hook being movable inside a portion of the implant.

A closely related fourth aspect of the invention provides an implant deliverable by catheter for treating prolapse of an atrioventricular valve (for example, by shortening a functional length of a native chordae), the implant comprising structure configured to divert a portion of the native chordae along a circuitous path, at least a majority of the circuitous path defined by the implant.

A closely related fifth aspect of the invention provides an implant deliverable by catheter for treating prolapse of an atrioventricular valve (for example, by shortening a functional length of a native chordae), the implant comprising a first portion and a second portion, the second portion being configured to deploy around and/or with respect to, the first portion when the implant changes from the non-deployed configuration to the deployed configuration.

A closely related sixth aspect of the invention provides an implant deliverable by catheter for treating prolapse of an atrioventricular valve (for example, by shortening a functional length of a native chordae), the implant comprising a first portion and a second portion, the second portion being configured to fold around and/or with respect to, the first portion when the implant changes from the non-deployed configuration to the deployed configuration.

A closely related seventh aspect of the invention provides an implant deliverable by catheter for treating prolapse of an atrioventricular valve (for example, by shortening a functional length of a native chordae), the implant comprising a structure defining a path for accommodating a native chordae, the structure configured to compress the path and/or compress around the path, when the implant changes from the non-deployed configuration to the deployed configuration.

A closely related eighth aspect of the invention provides an implant deliverable by catheter for treating prolapse of an atrioventricular valve (for example, by shortening a functional length of a native chordae), the implant comprising a structure defining a path for accommodating a native chordae, the structure configured to compress the native chordae tendineae and/or compress around the native chordae tendineae, when the implant changes from the non-deployed configuration to the deployed configuration.

A closely related ninth aspect of the invention provides an implant deliverable by catheter for treating prolapse of an atrioventricular valve (for example, by shortening a functional length of a native chordae), the implant comprising an elongate clamping surface configured to provide spread clamping contact along a native chordae tendineae, the elongate clamping surface being curved, linear or curvilinear.

A closely related tenth aspect of the invention provides an implant deliverable by catheter for treating prolapse of an atrioventricular valve (for example, by shortening a functional length of a native chordae), the implant comprising comprises a curved surface defining an at least partly curved circuitous path for receiving the native chordae tendineae. The curved surface may optionally define a majority of the circuitous path as a curved path.

A closely related eleventh aspect of the invention provides an implant deliverable by catheter for treating prolapse of an atrioventricular valve (for example, by shortening a functional length of a native chordae), the implant comprising a first part pivotally coupled to a second part, the first and second parts collectively defining a circuitous path for the native chordae. The portions of the first and second parts defining the circuitous path may optionally define surfaces that are curved, linear of curvilinear.

A closely related twelfth aspect of the invention provides an implant deliverable by catheter for treating prolapse of an atrioventricular valve (for example, by shortening a functional length of a native chordae), the implant comprising a structure defining a circuitous path for a native chordae that alternates between opposite portions and/or sides of the implant. For example, the structure may define a buckle-like structure for guiding a native chordae on a meandering and/or undulating path.

A closely related thirteenth aspect of the invention provides an implant deliverable by catheter for treating prolapse of an atrioventricular valve (for example, by shortening a functional length of a native chordae), the implant comprise first and second portions having confronting surfaces for trapping a native chordae therebetween, at least one of the confronting surfaces of the first and second portions comprising at least one projecting tooth, and at least the other confronting surface comprising a recess for accommodating the tooth. Optionally, the confronting surfaces of both of the first and second portions comprise interfitting toothed profiles, optionally interlocking toothed profiles. In some embodiments, the confronting surfaces are curved. The first and second portions may define a curved circuitous path that meanders around toothed profiles along the length of the curve.

A closely related fourteenth aspect of the invention provides an implant deliverable by catheter for treating prolapse of an atrioventricular valve (for example, by shortening a functional length of a native chordae), the implant comprising a loop structure. The loop structure may optionally comprise a movable gate for admitting a native chordae to an interior space of the loop structure. Additionally or alternatively, the loop structure may optionally be configured to fold around a portion of the implant.

A closely related fifteenth aspect of the invention provides apparatus for treating prolapse of an atrioventricular valve (for example, by shortening a functional length of at least one native chordae), the apparatus comprising a plurality of implants, each attachable to the or a native chordae, and a delivery catheter having an accommodation region at or near a distal end, for accommodating the plurality of implants together. In some embodiments, at least two of the plurality of implants may be deployed generally concurrently by the delivery catheter, for example, for attachment to the same native chordae. Additionally or alternatively, in other embodiments, the implants may be deployed individually, for example, for attachment to different respective chordae and/or to different respective chordae portions.

A closely related sixteenth aspect of the invention provides apparatus for treating prolapse of an atrioventricular valve (for example, by shortening a functional length of at least one native chordae), the apparatus comprising a delivery catheter packaged in sterile packaging, the delivery catheter pre-loaded with at least one implant deployable from the catheter, for example, for attachment to a native chordae. Optionally, the catheter is pre-loaded with a plurality of implants.

A closely related seventeenth aspect of the invention provides an implant deliverable by catheter for treating prolapse of an atrioventricular valve (for example, by shortening a functional length of a native chordae), the implant comprising a mesh and/or braid structure, the structure configured to change shape to define, at least partly, a circuitous path for a native chordae.

A closely related eighteenth aspect of the invention provides an implant deliverable by catheter for treating prolapse of an atrioventricular valve (for example, by shortening a functional length of a native chordae), the implant comprising at least one part having a shape that is concave in a first direction, and convex in a second direction generally perpendicular to the first direction.

A closely related nineteenth aspect of the invention provides an implant deliverable by catheter for treating prolapse of an atrioventricular valve (for example, by shortening a functional length of a native chordae), the implant comprising first and second parts that, in a deployed condition of the implant, curve around each other, optionally in first and second different directions (e.g. perpendicular directions). The implant may have a generally bead or bulb shape. The shape may optionally be generally spherical.

A closely related twentieth aspect of the invention provides an implant deliverable by catheter for treating prolapse of an atrioventricular valve (for example, by shortening a functional length of a native chordae), the implant having a generally bead shape, at least in a deployed configuration of the implant. The bead shape may optionally be generally spherical.

A closely related twenty-first aspect of the invention provides an implant deliverable by catheter for treating prolapse of an atrioventricular valve (for example, by shortening a functional length of a native chordae), the implant comprising, at least in a deployed configuration, one or more surfaces defining at least partly a channel for accommodating and pressing on a native chordae extending therethrough. At least one surface may comprise and/or be coated with and/or carry (e.g. as a liner), a compressible and/or flowable material able to conform at least partly around the profile of the native chordae while frictionally engaging against the native chordae. Example materials include silicone, polyurethane foam, and biocompatible rubbers. Such an arrangement can provide a secure yet atraumatic engagement with a native chordae.

A closely related twenty-second aspect of the invention provides a method of treating an atrioventricular cardiac valve, the method comprising introducing an implant to a target side in a heart using a delivery catheter, and attaching the implant to a native chordae to functional shorten the native chordae.

The catheter may be introduced using a transvascular route to the heart. In some embodiments, the transvascular route may be a transvenous transseptal route. In other embodiments, the transvascular route may be an arterial route.

The foregoing first to twenty-second aspects may be used independently of each other, or any two or more of the foregoing aspects may be combined together. All possible permutations and/or combinations of aspects are explicitly envisaged.

It will be appreciated from the above that the techniques of the invention can enable leaflet prolapse to be corrected while preserving native function of the sub-valvular apparatus. Shortening the functional length of a native chordae is also different from implanting an artificial chordae and adjusting the length of the artificial chordae during or after implantation. An artificial chordae is an artificial strand or filament that is less vulnerable to damage than would be a native chordae, especially a native chordae which has already stretched beyond its normal length resulting in leaflet prolapse. Artificial chordae are normally not attached to mobile leaflet regions, contrary to the native chordae. Special considerations apply in design of an implant for a native chordae, to avoid the implant from damaging or wearing the native tendon tissue of the chordae during the repeated opening and closing movements of the mobile leaflet portions from which native chordae extend.

Although certain features, ideas and advantages have been highlighted above, this is merely to aid understanding certain principles of the invention, without limiting the overall scope. Protection is claimed for any novel feature or idea described herein and/or illustrated in the drawings, whether or not emphasis has been placed thereon.

### Brief Description of the Drawings

Fig. 1 is a schematic sectional view of a heart ventricle illustrating a dysfunctional atrioventricular valve suffering from leaflet prolapse.
Fig. 2 is a schematic sectional view similar to Fig. 1, illustrating correction of prolapse by shortening the functional length of a native chordae tendineae.
Figs. 3a and 3b are schematic sectional views illustrating a first example of apparatus for treating prolapse.
Fig. 4 is a schematic sectional view showing the tip of the apparatus of Fig. 3 in more detail, carrying the implant in its non-deployed configuration, with the hook of the apparatus retracted ready to deploy the implant.
Fig. 5 is a schematic side view illustrating the implant of the first example in its deployed configuration attached to a native chordae.
Fig. 6 is a schematic sectional view of a second example of implant in its deployed configuration.
Fig. 7 is a schematic sectional view of a third example of implant.
Fig. 8 is a schematic plan view of a fourth example of implant.
Fig. 9 is a schematic side view of the fourth example of implant.
Fig. 10 is a schematic side view of a fifth example of implant in a deployed configuration.
Fig. 11 is a schematic side view of the fifth example of implant in a non-deployed configuration.
Fig. 12 is a schematic side view of the fifth example of implant attached to a native chordae.
Fig. 13 is a schematic side view of a sixth example of implant in a deployed configuration.
Fig. 14 is a schematic side view of a seventh example of implant in a non-deployed configuration.
Fig. 15 is a schematic side view of the seventh example of implant in a deployed configuration attached to a native chordae.
Fig. 16 is a schematic sectional view of an eighth example of implant carried by a catheter in a non-deployed configuration.
Fig. 17 is a schematic side view illustrating eversion of the implant between its different configurations.
Fig. 18 is a schematic sectional view of the eighth example of implant in its deployed configuration attached to a native chordae.
Fig. 19 is a schematic side view of a ninth example of implant in a non-deployed configuration.
Fig. 20 is a schematic side view of the ninth example of implant in a deployed configuration attached to a native chordae.
Fig. 21 is a schematic transverse view of the ninth example of implant.
Fig. 22 is a schematic side view of a tenth example of the implant in a non-deployed, open configuration, carried by a catheter
Fig. 23 is a schematic side view of the tenth example of the implant in a deployed, closed configuration, carried by a catheter.
Fig. 24 is a schematic sectional view similar to Fig. 23, but showing attachment to a native chordae.
Fig. 25 is a schematic side view of the tenth example of implant released from the catheter.
Fig. 26 is a schematic perspective view of an eleventh example of implant in a partly deployed (folded) configuration.
Fig. 27 is a schematic perspective view of the eleventh example in a non-deployed (unfolded) configuration.
Fig. 28 is a schematic sectional view of the eleventh example deployed (fully folded) configuration attached to a native chordae.
Fig. 29 is a schematic sectional view of a twelfth example of implant in a non-deployed configuration in a delivery catheter.
Fig. 30 is a schematic sectional view of the twelfth example attached to a native chordae.
Fig. 31 is a schematic sectional view of a thirteenth example of implants in a non-deployed configuration in a delivery catheter.
Fig. 32 is a schematic side view of a fourteenth example of implant comprising first and second parts nesting together.
Fig. 33 is a schematic sectional view of the fourteenth example of implant in a deployed configuration.
Fig. 34 is a schematic sectional view of the fourteenth example of implant in a non-deployed configuration attached to a delivery catheter.
Fig. 35 is a schematic sectional view of the fourteenth example when attached to a native chordae and released from the delivery catheter.
Fig. 36 is a schematic section view illustrating the delivery catheter for the fourteenth example.
Fig. 37 is a schematic sectional view of a fifteenth example of implant in a deployed configuration attached to a native chordae.

### Detailed Description of Preferred Embodiments

Non-limiting embodiments of the invention are now described by way of example, with reference to the accompanying drawings. In the drawings, the same or equivalent features are denoted by the same reference numerals whether or not described explicitly.

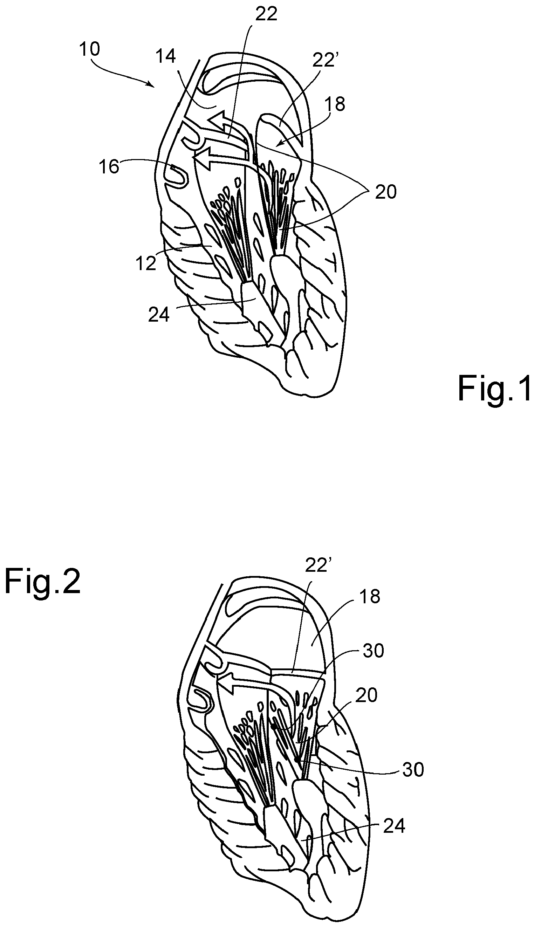

Referring to Fig. 1, a portion of a heart 10 is illustrated having a ventricle 12, an atrium 14, an outflow tract valve 16 leading to an artery from the heart, and an atrio-ventricular valve 18 between the ventricle 12 and atrium 14. The ventricle 12 may be a left ventricle, the outflow valve 16 an aortic valve, and the atrio-ventricular valve 18 a mitral valve. Alternatively, the ventricle 12 may be a right ventricle, the outflow valve 16 a pulmonary valve, and the atrio-ventricular valve 18 a tricuspid valve.

Chordae tendineae 20 (hereafter chordae) connect the leaflets 22 of the atrio-ventricular valve 18 to papillary muscles 24 in the ventricle 12, to control range of movement of the leaflets 22. In the example of Fig. 1, at least one leaflet 22' of the atrio-ventricular valve 18 is illustrated to suffer from prolapse, in which the chordae 20 do not restrain the leaflet 22' properly within the ventricle during systole, allowing the leaflet 22' to be pushed out of its correct position and at least partly into the atrium. Leaflet prolapse is a major cause of valve insufficiency and atrio-ventricular regurgitation, as illustrated by the white blood flow arrows.

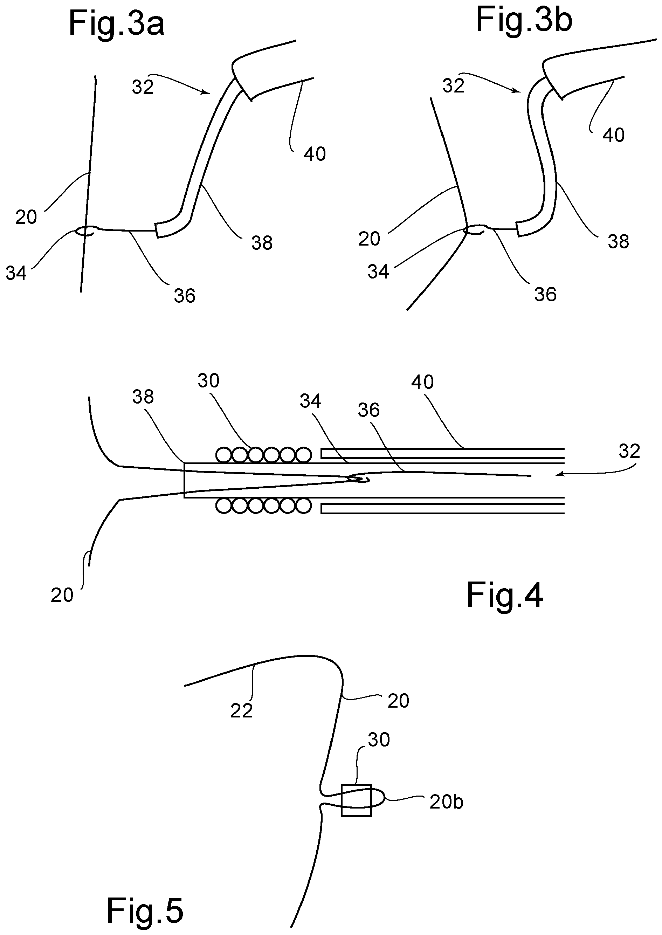

Figs. 2 to 4 illustrate one technique of the present invention to treat prolapse by a transcatheter procedure. An implant 30 (optionally multiple implants 30) is delivered by a delivery catheter (32 in Fig. 3) into the ventricle 12. The implant 30 is configured to attach to a native chordae 20, optionally an individual native chordae 20, to shorten the functional length of the native chordae 20. By shortening the functional length, the chordae 20 can better restrain the leaflet 22', correcting against prolapse, and restoring or improving valve function (as indicated by the white blood flow arrow in Fig. 2).

The catheter may be introduced to a target site in the ventricle 12 and/or in the atrio-ventricular valve 18 by various access routes, including transvascular routes. For example, the catheter may be introduced via an arterial route through the outflow valve 16, for example, a transfemoral or transhumeral/transsubclavian route. Additionally or alternatively, the catheter may penetrate through the femoral vein and/or pass through the septum between left and right atria 14, namely a transseptal route.

Various examples of implant are described below. In some embodiments, the implant 30 is configured to change from a non-deployed configuration for delivery, to a deployed configuration for attachment to a native chordae. The implant 30 may be self-deploying when released from the delivery catheter, and/or the catheter may apply a deploying force to the implant to change the configuration. The implant 30 may optionally be made of a shape-memory material and/or super-elastic material, for example, a shape-memory alloy. One such alloy may comprise at least nickel and titanium, for example, nitinol. Other suitable materials include one or more of titanium, PEEK, and/or pyloric carbon.

The implant 30 may define a circuitous path that shortens the function length of a native chordae by at least 1mm, optionally at least 2mm, optionally at least 3mm, optionally at least 4mm, optionally at least or about 5mm, optionally at least 10mm, optionally 15-20 mm in some cases. Optionally, multiple implants 30 may be attached to the same chordae to accumulate the shortening effects and achieve greater degrees of shortening than a single implant. In some embodiments, the implant is lightweight so as to avoiding adding significant load to the native chordae, especially should a diseased chordae rupture at one point. Also, in some embodiments, the implant has a streamlined profile in the implanted or deployed configuration.

The implant 30 may optionally be supplied pre-attached to, or pre-loaded in, the delivery catheter as a sterile assembly. The assembly may optionally be supplied packaged in sterile packaging. Alternatively, the implant may be supplied separately from the catheter, and attached to and/or loaded in the catheter shortly before the patient procedure.

Referring to Figs. 3 to 5, the catheter 32 may comprise a hook 34 configured for hooking a native chordae 20 to pull the native chordae 20 proximally, ie. in a proximal direction of, or with respect to, the catheter 32. In the illustrated example, the catheter comprises a plurality of tubes nested one within another. The hook 34 is carried by a shaft 36 that is extendable from and/or retractable into the interior bore of a hollow carrier tube 38 carrying the implant 30. The carrier tube 38 is itself slidable within the bore of a pusher tube 40. In this example, the implant 30 has the form of a narrow-bore elongate coil. In a non-deployed configuration, the coil mounted outside the carrier tube 38 is held in a widened state. When released, the coil self-contracts to its narrow bore, deployed state.

In use (Fig. 3a), the catheter 32 is advanced to a target site in the vicinity of the native chordae 20 to be shortened, and the hook 34 is extended to hook the native chordae 20. The hook 32 is retracted (fig. 3b) to divert a portion 20a of the hooked chordae 20 towards the carrier tube, and ultimately into the carrier tube 38 (Fig. 4). Relative retraction of the carrier tube 38 and/or advancement of the pusher tube 40 pushes the coil implant 30 progressively off the free end of the carrier tube 38 and on to the chordae portion 20a.

Referring to Fig. 5, the implant coil 30 contracts towards its narrow bore configuration, clamping the chordae portion 20a within its interior. A portion 20b of the chordae forms a free loop at one end of the coil implant 30, but a majority of the chordae portion 20a is trapped in a circuitous hairpin path defined by the bore of the coil implant 30. The clamping force is distributed over a relatively long majority of the chordae portion 20a.

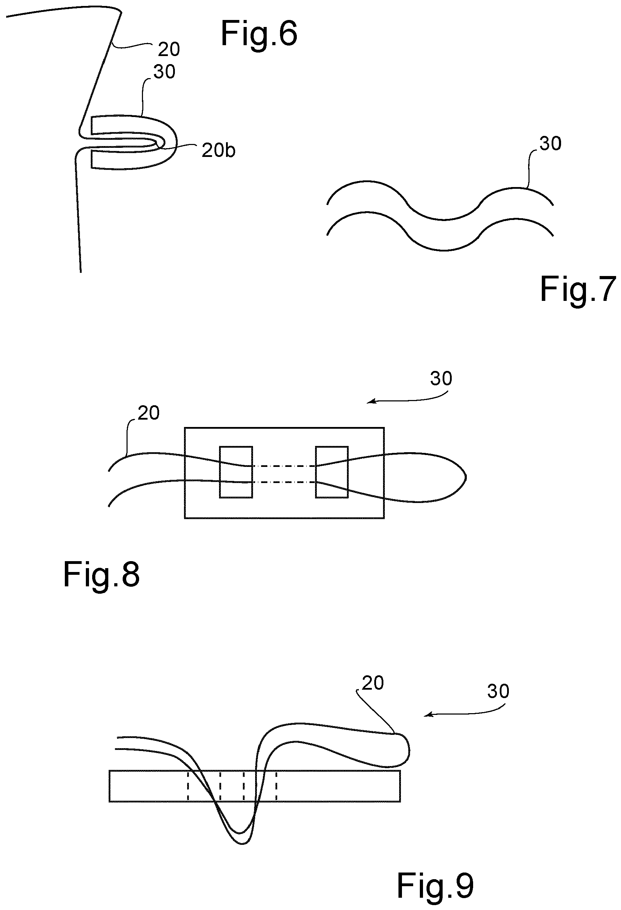

Referring to Fig. 6, a second embodiment of implant 30 similar to the first example, comprises a self-closing clip that traps at least a majority, optionally substantially the entirety, of the diverted chordae portion 20a with its interior. The interior defines a circuitous path also in the form of a hairpin.

Although in Figs. 5 and 6, the implant has a generally straight configuration, referring to Fig. 7, a third example of implant 30 may have a curvilinear configuration to define a wavy and/or tortuous path within the implant 30. A tortuous path can provide a longer length of circuitous path, and hence increase the shortening effect of the implant 30, as well as help prevent the chordae portion 20a from slipping out of the implant 30.

Figs. 8 and 9 illustrate a fourth example similar to Fig. 7, except that the implant 30 has a buckle profile, through which a native chordae can be pulled. Figs. 8 and 9 illustrate a folded chordae pulled through creating a double pass. The buckle like implant 30 could be attached to the chordae using the same principle shown in the Fig. 4.

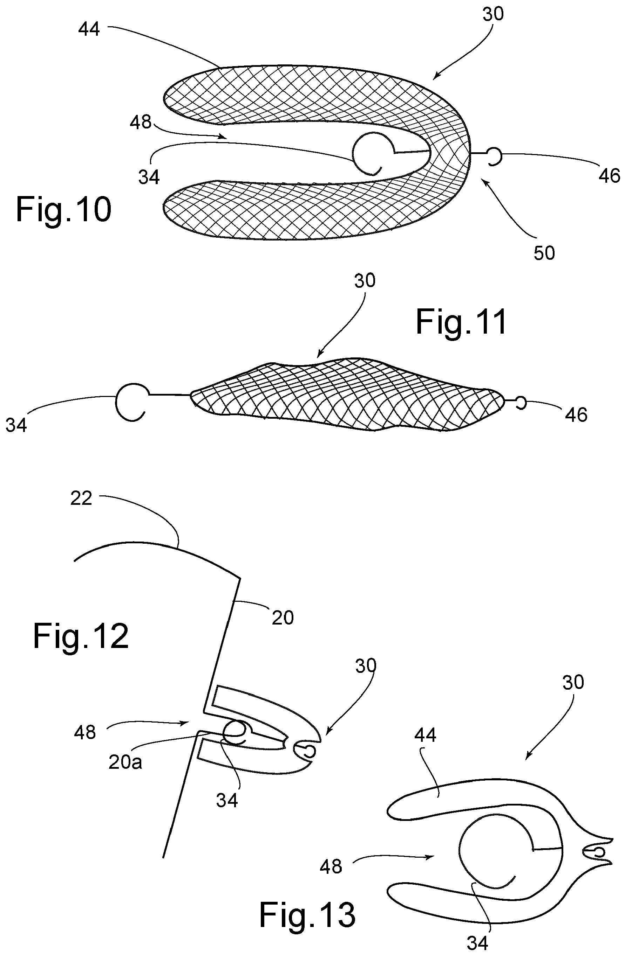

Figs. 10-12 illustrate a fifth example of implant 30 in which the hook 34 forms part of the implant. The implant comprises a (e.g. continuous) wire mesh 44 made, for example, of shape-memory material. The hook 34 is integrally mounted to one side, and a handle or grip 46 in the form of a secondary hook (here shown as an example while other connecting/disconnecting principles could be used) is mounted generally opposite. The handle (e.g. secondary hook) 46 enables the implant 30 to be attached to and manipulated by the catheter 32. Alternatively, the implant comprises a continuous wireform loop.

In its natural, deployed configuration (Fig. 10), the wire mesh and/or wireform loop defines a cup and/or channel 48 around the hook 34, with the hook 34 generally at the base of the cup/channel 48. The secondary hook 46 can optionally be accommodated in a smaller recess 50 intended to shroud any sharp or abrupt edges of the secondary hook 46 (figs. 12 and 13).

In use, the implant 30 is unfolded to a generally low profile, elongated, non-deployed configuration (Fig. 11) in which the implant is delivered to a target site by a catheter (not shown). The implant 30 is restrained in the non-deployed configuration by means of a constraining sheath, for example. In the non-deployed configuration, the hook 34 is exposed for hooking on to a native chordae portion 20a.

Referring to Fig. 12, when the constraining sheath is removed, the implant 30 reverts to its deployed configuration, deploying and/or folding around the hook 34 and pulling the native chordae portion 20 to divert the chordae portion 20a into the cavity of the channel 48, thereby shortening the functional length of the chordae 20.

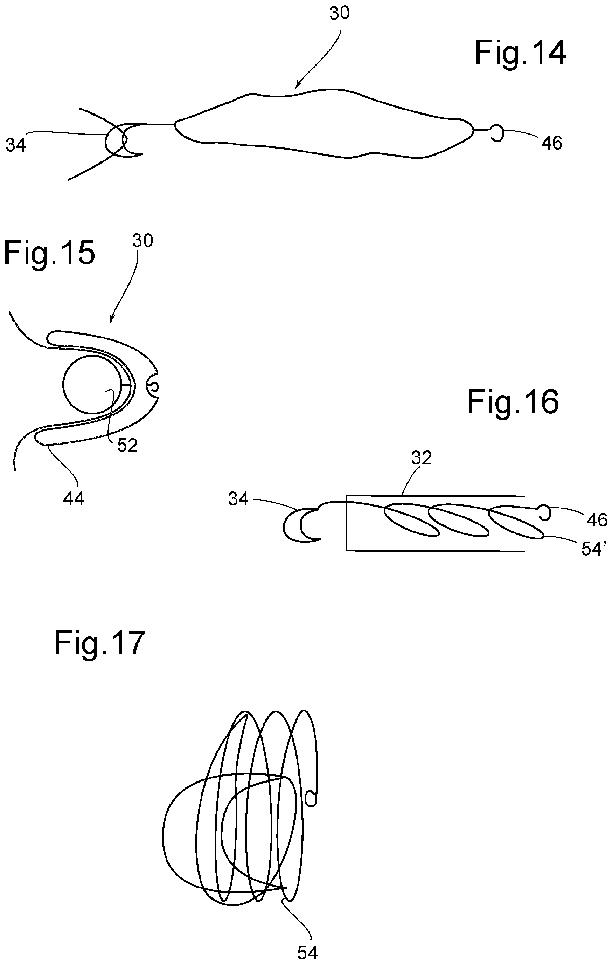

Figs. 13 illustrates a sixth example similar to the fifth example, but in which the wire mesh and/or wireform 44 has a more streamlined or curved shape folded around the hook 34, and defining a narrower and more rounded mouth to the channel 48. In Figs. 12 and 13, and also Figs. 14-15 described below, the mesh detail is omitted to avoid cluttering the drawing.

Figs. 14 and 15 illustrate a seventh example similar to the fifth and sixth examples above, but in which the hook 34 has a three-dimensional profile. For example, the hook 34 provides a concave hook recess in a lateral and/or transverse direction to allow lateral/transverse hooking of a chordae (Fig. 14). Additionally, the hook 34 has a convex profile in a perpendicular direction to define a curved hub surface 52 (Fig. 15). Alternatively, the hook 34 may be configured to change configuration as the implant 30 changes from the non-deployed configuration (Fig. 14) to the deployed configuration (Fig. 15). The hook 34 has arms that close to form a curved hub surface 52 around which the deviated chordae portion 20a extends, and around which the wire mesh and/or wireform 44 of the implant 30 folds. By using a curved surface 52, the circuitous path around the hub can be lengthened compared to a straight hairpin path. Also, the chordae is clamped over a relatively long contact surface. Additionally or alternatively, the central portion of the hook 34 could be made thicker as compared to the hooks from Figs. 10 to 13, to achieve a similar effect.

Figs. 16-18 illustrate an eighth example of implant 30 comprising a hook 34 similar to the hook of the sixth example, having a three-dimensional profile and/or configured to change configuration to a curved hub surface 52 when the implant is deployed. A coiled shell 54 extends from and cups around the hook/hub surface 52 to define a circuitous deviation path for a portion of a native chordae.

In the non-deployed configuration of the implant 30, the coiled shell 54 is pulled proximally away from the hook 34 to an everted condition 54', as illustrated in Fig. 16, and constrained within a sheath of the delivery catheter 32. The hook 34 is exposed to facilitate hooking on a native chordae.

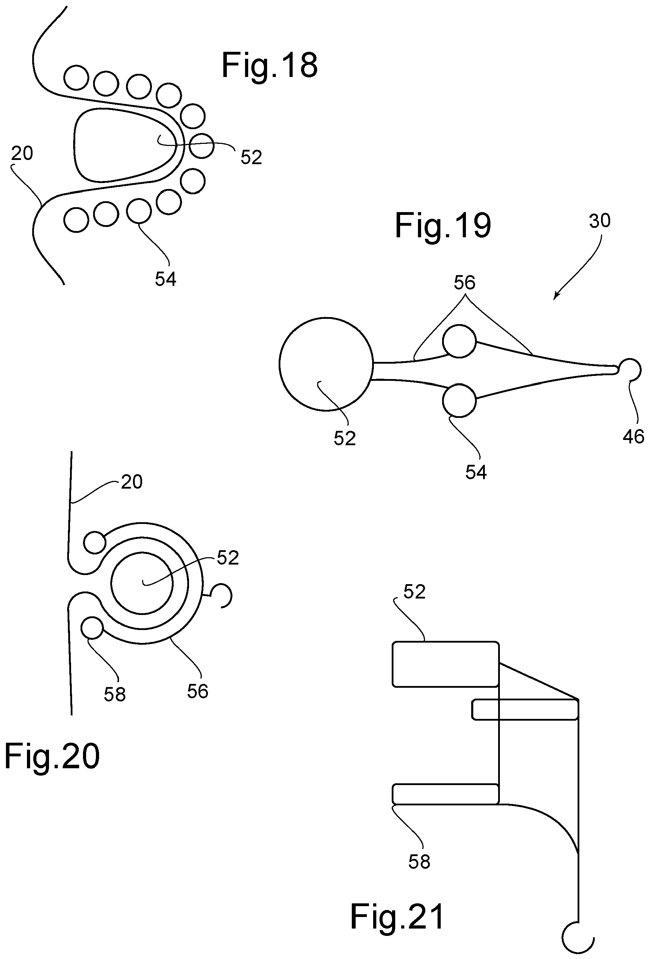

As depicted schematically in Fig. 17, when the everted coil 54' is released from the sheath, the coil 54 reverts distally to its original configuration. Referring to Fig. 18, the coil 54 cups around the hook 32/hub 52, and clamps the native chordae in a circuitous path extending around the curved surface of the hub 52.

Figs. 19-21 illustrate an ninth example of implant 30 comprising a hub 52 to which are coupled first and second arms 56 carrying lobes or posts 58. As can be seen in Fig. 21, the hub 52 and the lobes/fingers 58 have a transverse dimension greater than that of the arms 56, to define surfaces over which a native chordae is guided on a circuitous path. In a non-deployed configuration, the arms 56 are pulled proximally away from the hub 52 into a delivery catheter sheath (not shown), allowing the hub 52 to be hooked around a native chordae 20. When the implant is released, the arms 56 fold with respect to the hub 52, e.g. fold around the hub 52, such that the posts 58 divert the native chordae on a circuitous path around the hub 52. The implant further comprises a secondary hook 46 similar to the preceding embodiments.

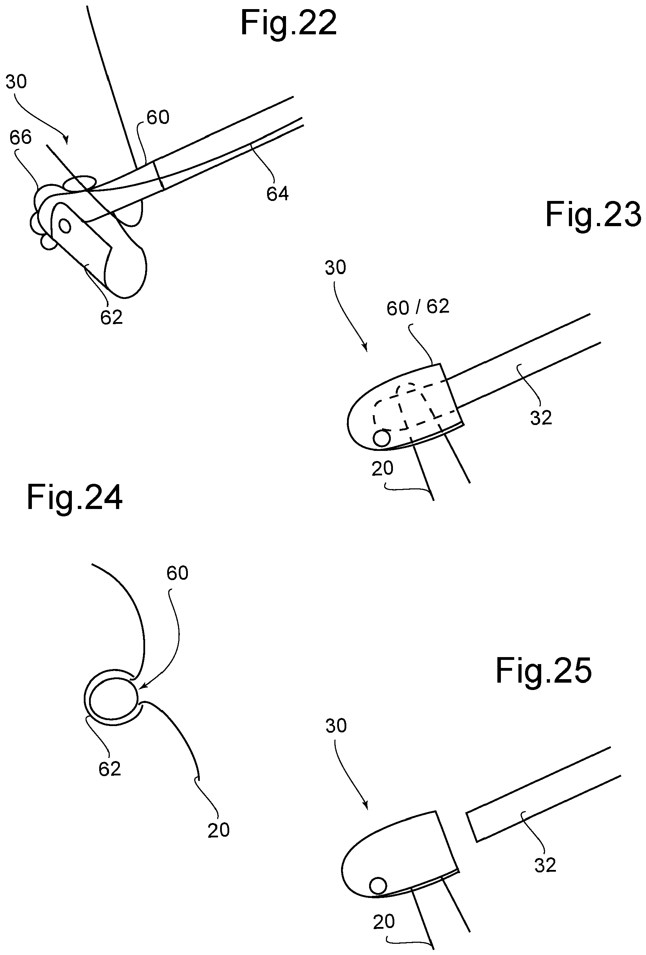

Figs. 22-25 illustrate a tenth example of implant 30 comprising first and second pivoting parts 60 and 62 controlled by means of an actuator 64 of the delivery catheter 32. The first and second parts 60 and 62 are arranged one nested within another, and have matching curved profiles to define a circuitous path therebetween around the tip of the inner part. The inner part acts as a curved head around which the outer part can pivot. In a non-deployed configuration of the implant 30, for delivery to a target site (Fig. 22), the outer part (e.g. the second part 62) is pivoted open away from the inner part to allow a portion of a native chordae to enter between the two parts. By actuating the implant 30 into the deployed configuration (Figs. 23 and 24), the outer part pivots into close engagement with the inner part, thereby diverting the chordae along the circuitous path defined between the two parts 60 and 62. The first and second parts 60 and 62 can be retained in the deployed configuration by any of: friction; and/or a resilient bias (e.g. spring 66 in Fig. 22); and/or a locking mechanism (not shown). After the implant 30 has been attached to the native chordae 20, the delivery catheter 32 is detached from the implant and withdrawn (Fig. 25).

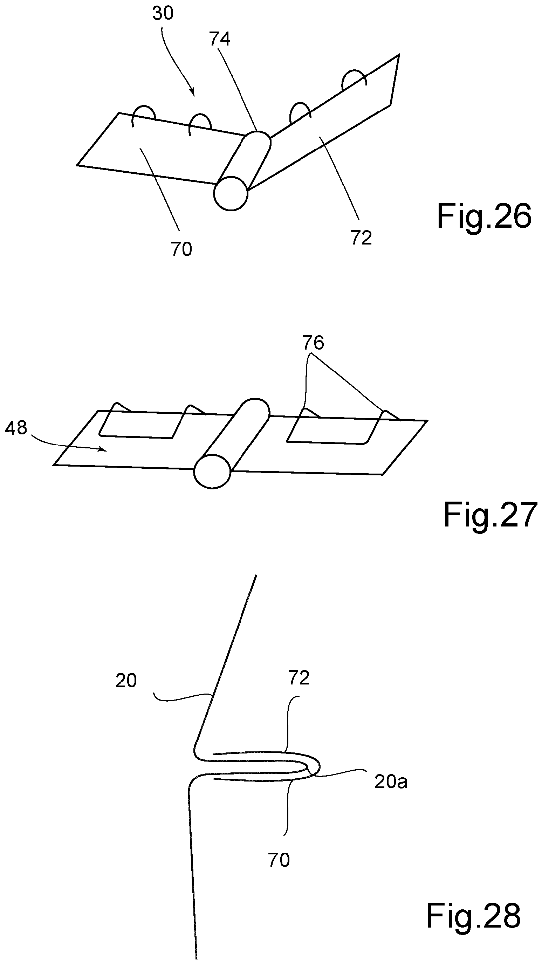

Figs. 26-28 illustrate an eleventh example of implant 30 comprising first and second limbs 70 and 72 that pivot relative to each other about a transverse hinge axis 74. The limbs 70 and 72, and/or trapping fingers 76 carried by the limbs 70 also fold over in a longitudinal direction generally perpendicular to the hinge axis 74 to define a clamping channel for receiving the native chordae 20. Once clamped, the first and second limbs 70 and 72 are pivot closed like jaws, to define a multiple-folded clamp (Fig. 28), defining a generally hairpin circuitous path. The implant 30 may be held in the deployed configuration by any of: friction; and/or a resilient bias (not shown); and/or a locking mechanism (not shown).

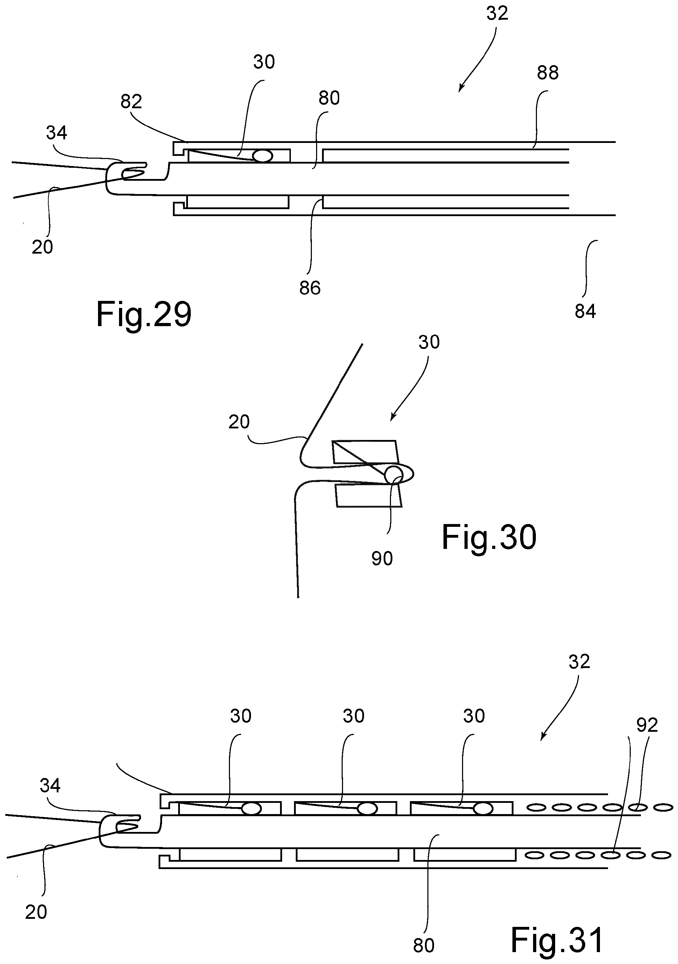

Figs. 29 and 30 illustrate a twelfth example of implant 30 and delivery catheter 32, which are similar in some respects to those of the first example (Figs. 3-5). The implant 30 comprises a self-contracting tube or coil configured to self-contract from an expanded condition for delivery (Fig. 29) to a contracted configuration (30) in which the tube accommodates a portion of a native chordae 20 in a hairpin shape. Also, the delivery catheter 32 comprises a hook 34 that is extendable to hook on a native chordae 20 (Fig. 29), and retractable to pull the native chordae proximally into the implant 30.

However, the twelfth example has additional features. Referring to Fig. 29, instead of requiring a carrier tube to hold the implant 30 in its expanded configuration for delivery, the implant 30 is supported directly by a shaft 80 on which the hook 34 is mounted. The shaft 80 is dimensioned to hold the implant 30 in its expanded non-deployed configuration for delivery. The shaft 80 is extendable to expose the hook 34, and retractable to pull the hook 34 into the interior of the catheter 32. The catheter 32 further includes stops to restrain the implant 30 axially during movement of the shaft 80, despite friction between the implant 30 and the shaft 80. In this example, a distal stop comprises an inwardly turned lip 82 of an outer cover tube 84, and a proximal stop comprises a blocking end surface 86 of an intermediate tube 88 within which the shaft 80 slides.

In use, the shaft 80 is extended to expose the hook 42 for hooking a native chordae 20. The shaft 80 is then retracted to pull the native chordae into the interior of the catheter 32. Once the shaft 80 and hook 42 have cleared the implant 30, the implant 30 is free to contract to its deployed configuration (Fig. 30) to attach around the portion of native chordae that has been pulled through the implant. The deployed configuration has a smaller diameter in which the implant is no longer restrained by the lip 82, and so is free to separate from the catheter 32. The catheter 32 can be withdrawn away from the deployed implant 30, or the shaft 80 can be advanced distally to eject the implant 30 using the hook 34 as a pusher.

A further feature of the implant 30 of this example is provision of a self-deploying projection 90. In the non-deployed configuration (Fig. 29), the projection 90 is folded into the wall of the implant 30. In the deployed configuration (Fig. 30), the projection 90 deploys towards and/or across the interior passage of the implant 30, to define a surface behind the tight bend of the native chordae, thereby preventing the native chordae 20 from pulling out of the implant once the projection 90 has deployed. In the illustrated example, the projection 90 extends diagonally from one end. In an alternative embodiment, the projection could swing more transversely from the opposite end.

Fig. 31 illustrates a thirteenth example similar to the twelfth example above. The main difference is that the catheter is configured to accommodate multiple implants 30 loaded in line with each other at the tip. The implants 30 are deployed by manipulation of the shaft 80. For example, multiple implants 30 may be attached to the same native chordae 20 by retracting the shaft 80 sufficiently proximally to clear the appropriate number of implants 30 to be deployed together. Alternatively, each implant 30 can be deployed individually by retracting the shaft 80 only so far to clear the distal-most implant 30. The distal-most implant 30 contracts on to the chordae 20 and separates from the catheter 32, while the other implants 30 remain stocked within the catheter 32 and held in the non-deployed condition by the shaft 80. A spring-biased pusher 92, for example, a coil spring, urges the implants 30 distally to replace the deployed implant at the tip ready for a subsequent deployment. Such a catheter 32 allows multiple implants 30 to be attached, without having to withdraw and reinsert the catheter each time. Such a multiple use catheter 32 can greatly increase the versatility of use, and reduce the duration and invasiveness of the procedure.

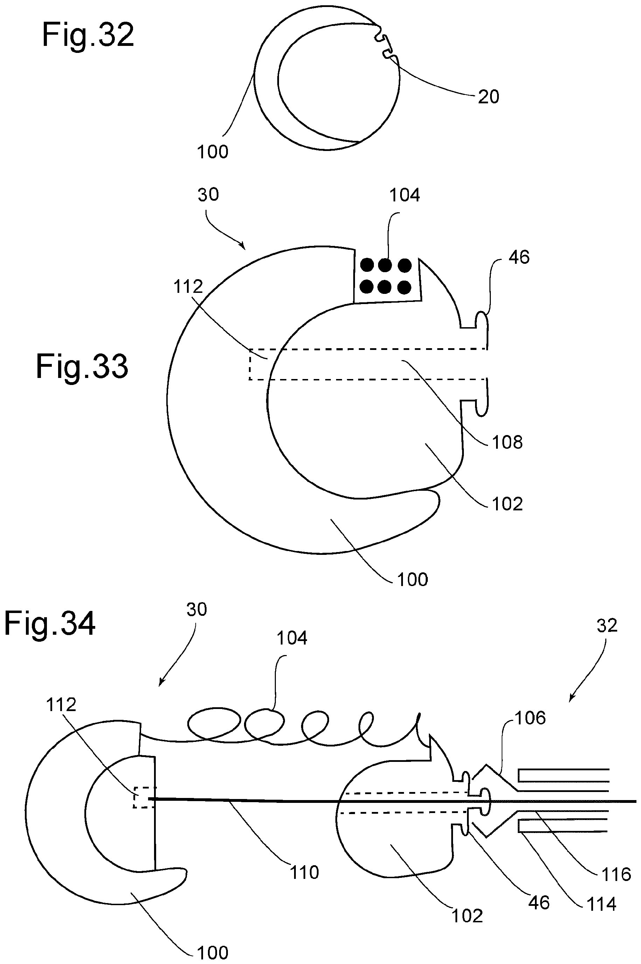

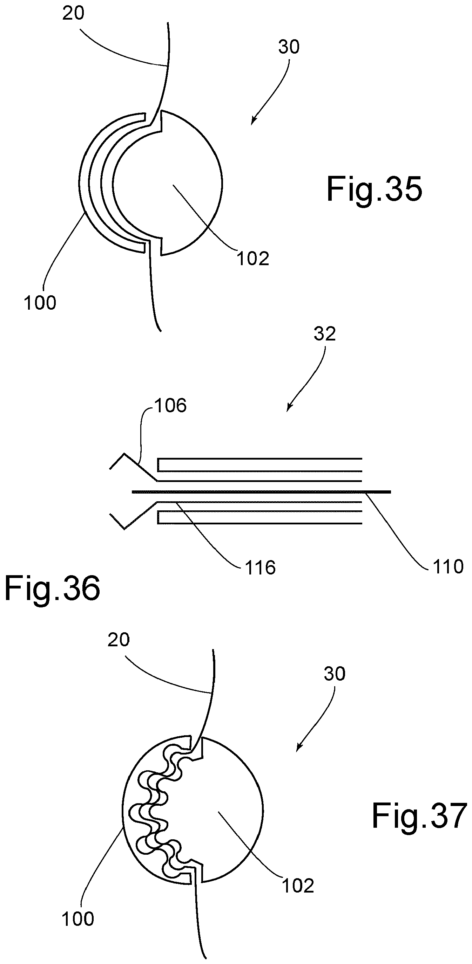

Figs. 32 to 36 illustrate a fourteenth example similar to the tenth example above, in that the implant 30 comprises complementary first and second parts 100 and 102 having curved profiles to define a circuitous path therebetween. In the present example, the first and second parts 100 and 102 are movable slidably with respect to each other. A spring 104 biases the parts 100 and 102 towards each other into the deployed configuration trapping a portion of the native chordae 20 between the parts 100, 102 (Fig. 35).

One part 102 comprises a handle 46 in the form of a nipple that can be gripped by gripper jaws 106 of the catheter 32, and a through bore 108 through which a pusher rod 110 of the catheter 32 can be advanced to push the parts 100 and 102 apart. The other part 100 optionally comprises a socket 112 for receiving the tip of the pusher rod 110. The through bore 108 is aligned centrally with the nipple 46. The catheter 32 comprises an outer sleeve 114 within which a hollow tube 116 carrying the gripper jaws 106 is slidable. The gripper jaws 106 are biased open but, by retracting the tube 116 with respect to the sleeve 114, the jaws 106 close inwardly to enable the nipple 46 to be gripped by the catheter 32 until the implant 30 is ready to be released.

The pusher rod 110 is slidable within the bore of the tube 116. In use, by advancing the pusher rod 110 while holding the tube 116 retracted to grip the nipple 46, the parts 100 and 102 of the implant are pushed apart against the bias of the spring 104. To attach the implant 30 to a native chordae 20, the parts 100 and 102 are hooked around the native chordae 20, and the pusher rod 110 retracted. The spring 104 pulls the parts 100 and 102 towards each other, to trap the native chordae 20 in a circuitous path between the curved profiles of the parts 100 and 102. Should the operator physician desired to adjust the position of the implant 30, this is possible while the implant 30 is still attached to the jaws 106. The pusher rod 110 may be advanced again to open the parts 100 and 102, and allow repositioning. Once the implant 30 has been correctly positioned and firmly attached, the tube 116 can be advanced to allow the jaws 106 to spring open, and release the nipple 46 of the implant. Thereafter, the catheter is withdrawn.

As best seen comparing Figs. 32 and 35, in the illustrated example, the parts 100 and 102 define a bulb or ball shape. The parts 100 and 102 may curve around each other in different, e.g. generally perpendicular, directions, for example, in a similar manner to the curvilinear surface contouring of a tennis ball, or in a similar manner to a person crossing the palms of his or her hands together. Such a form can assist in self-alignment of the parts 100 and 102 as they come together, while also providing a smooth outer surface. It can also provide complementary hook-style surfaces on both parts 100 and 102, acting in different directions. In other examples, the parts 100 and 102 could have more of a male-female configuration, such that one part 102 fits into the convex hollow of the other part 100.

In the fourteenth example, the confronting surfaces of the first and second parts 100 and 102 are generally smoothly curved with a large radius of curvature defining the circuitous path. Fig. 37 illustrates a fifteenth example in which the confronting surfaces have interlocking and/ortoothed profiles. Such profiles increase the length of the circuitous path, by additional meandering, compared to smooth profiles. Additionally or alternatively, the profiles can define a snap-fit engagement. Snap-fitting may be due to the angle of the teeth not all being aligned with the direction of movement. Alternatively, the some of the teeth may be configured to be oversize with respect to the mouth of the confronting recess, thereby forming a snap-fit as the teeth ride into the recesses. Snap-fitting can add a further degree of security to the attachment already provided by the spring 104.

In all of the preceding embodiments, the different parts of the catheter 32 may optionally have a fixed rotational alignment. A fixed rotational alignment may be implemented by, for example, mechanical keying, such as non-round surfaces of nested parts. For example, the nested parts may have an oval, or polygonal (e.g. triangular or square) cross-section shape. Alternatively, where desired, one or more nested parts may be rotatable with respect to each other.

Although some embodiments illustrate a handle 46 on the implant in the form of a hook, and other embodiments illustrate a handle 46 in the form of a nipple that can be gripped by jaws, these example forms of handle may be interchanged in any example above, and other forms of handle are also envisaged.

Although not illustrated specifically in the drawings, any of the above embodiments may optionally comprise at least one surface that comprises and/or is coated with and/or carries (e.g. as a liner), a compressible and/or flowable material able to conform at least partly around the profile of the native chordae while frictionally engaging against the native chordae. Example materials include silicone, polyurethane foam, and biocompatible rubbers. Such an arrangement can provide a secure yet atraumatic engagement with a native chordae.

It will be appreciated that the foregoing description is merely illustrative of preferred examples, and that many modifications and equivalents may be used within the scope and principles of the invention.

## Claims

1. Apparatus for treating prolapse of an atrioventricular valve, the apparatus comprising an implant (30) deliverable by catheter (32), the implant configured to attach to a native chordae tendineae (20) and to shorten the functional length of the native chordae tendineae.

2. Apparatus according to claim 1, wherein the implant (30) is attachable to an individual native chordae tendineae (20) and configured to shorten the functional length of the respective individual chordae tendineae.

3. Apparatus according to any preceding claim, further comprising a catheter (32) for delivering the implant to a target site in the heart, and a hook (34; 52) configured to hook the native chordae tendineae, and to pull the native chordae tendineae in a proximal direction with respect to the catheter.

4. Apparatus according to claim 3, wherein the hook (34; 52) is positioned internally with respect to a portion of the implant (30).

5. Apparatus according to claim 3 or 4, wherein the hook (34; 52) is moved inside a portion of the implant (30).

6. Apparatus according to claim 3, 4 or 5, wherein the hook (34; 52) forms part of the implant (30).

7. Apparatus according to claim 3, 4 or 5, wherein the hook (34) forms part of the catheter (32).

8. Apparatus according to any preceding claim, wherein the implant (30) is configured to divert a portion of the native chordae tendineae along a circuitous path, at least a majority of the circuitous path being defined by the implant.

9. Apparatus according to claim 8, wherein the implant (30) defines substantially the entirety of the circuitous path along which the portion of the native chordae tendineae is diverted.

10. Apparatus according to any preceding claim, wherein the implant (30) is configured to change configuration between a non-deployed configuration for delivery to a target site in the heart, and a deployed configuration for attaching to a native chordae tendineae.

11. Apparatus according to claim 10, wherein the implant (30) comprises a first portion (34; 52; 60) and a second portion (44; 54; 56; 58; 62), the second portion being configured to deploy and/or fold around the first portion when the implant changes from the non-deployed configuration to the deployed configuration.

12. Apparatus according to claim 10 or 11, wherein the implant (30) is configured to compress the native chordae tendineae and/or compress around the native chordae tendineae, when the implant changes from the non-deployed configuration to the deployed configuration.

13. Apparatus according to any preceding claim, wherein the implant (30) comprises an elongate clamping surface configured to provide spread clamping contact along a native chordae tendineae, the elongate clamping surface being any one or more of: curved; linear; curvilinear; meandering; defined by interlocking teeth.

14. Apparatus according to any preceding claim, wherein the implant (30) comprises a curved surface (52; 60) configured for defining an at least partly curved circuitous path for the native chordae tendineae, optionally an at least partly meandering curved circuitous path.

15. Apparatus according to any preceding claim, wherein the curved surface (52; 60) is configured to define a majority of the circuitous path as a curved path.
